# EUROPEAN PATENT APPLICATION

(11) **EP 2 407 092 A2**
(43) Date of publication of application: **18.01.2012**
(21) Application number: 11003725.6
(22) Date of filing: 07.01.2005
(51) Int. Cl.: A61B 5/00, G06Q 10/00, G06Q 50/00, G06F 19/00

(54) **Clinical data database system and method for a critical care and/or hospital environment**

(30) Priority: 09.01.2004 US 535390 P
(62) Divisional of application: 05708735.5
(71) Applicant: IMD-Soft, Ltd., 61581 Kiryat Atidim - Tel Aviv (IL)
(72) Inventor: David, Eran, Tel Aviv (IL)
(74) Representative: Hackett, Sean James

(57) **Abstract**

A system for capturing and maintaining a categorized flow of data in a clinical environment includes a distributed computer network, a plurality of data clusters dispersed over the network, a repository adapted to store data representative of the identity of patients, means for querying the repository to identify one of the data clusters associated with a particular patient and in response to such identification of the data cluster, allowing said particular patient or a user to query clinical data associated with the patient from said identified data cluster.

## Description

### CROSS-REFERENCE TO RELATED APPLICATIONS

This application claims priority of U.S. Provisional Patent Application Serial No. 60/535,390, filed on January 9, 2004.

### FILED OF THE INVENTION

This invention is in the field of medical information systems, and more particularly related to optimized methods and systems for capturing and maintaining a categorized flow of data in a clinical environment

### BACKGROUND OF THE DISCLOSURE

Clinical data is defined as data related to a patient. It includes data such as ADT, vital signs, analog waves, summaries, and the like. Clinical data is categorized by the patient; each patient has its own data. Clinical data is collected from various sources, such as medical devices, and hospital systems or may be manually entered by clinical staff. This can result in a huge amount of data flow that needs to be captured online. The clinical data, once captured, should be available for browsing, manipulating and query. In order to achieve high-performance for such issues (browsing/queries/manipulating),the data store (that is, the physical file itself where the data is kept) should have as small a size as possible (one that fits the machine hardware) and yet maintain all the patient data in the same store (to avoid the need for cross-file queries). Having multiple storages(for example, separate stores for each patient) can pose a maintenance problem. In addition, for the method of capturing and maintaining clinical data to be scalable, it should be possible to add additional hardware to handle parts of the clinical data flow. This invention provides a load balancing system and method for capturing clinical data, while keeping the number of physical data stores, and their size, low as possible.

### SUMMARY OF THE INVENTION

The system and method of the invention establishes a data base architecture that is particularly suited for entry, storage, management and access for clinical data in a critical care and/or more general hospital environment. The system and method of the invention is preferably operative over a geographically distributed network including multiple servers, data stores and user access terminals, but could alternatively be operative over a local area network. In one form, communication between network nodes can be effected over the internet.

In accordance with the invention, the database architecture includes a plurality of data stores referred to as Data Clusters. Each Data Cluster may store medical record data for predetermined number of patients. By way of example, the data stored for a patient at a Data Cluster may include heart rate data, body temperature data, diagnosis code data, demographic data as well as other data typically used in critical care and/or more general hospital environments. The amount of the data stored for various patients in a Data Cluster may vary from patient to patient. There may be clinical data that is representative of frequent data points over a short time period, or less-frequent data points over a long time period. Preferably, the Data Clusters are each associated with a particular server in the network. Each such server may be associated with zero, one or more Data Clusters.

A Repository is employed to maintain, in effect, a table of information which includes meta-data for each Data Cluster. The meta-data for a Data Cluster is representative of the identity of each patient for whom medical data records are stored on the Data Cluster, and of the type (and in some cases, the range) of data values stored at the Data Cluster.

The database architecture is accordingly structured so that a user of the system and method of the invention, may desire, for example, to obtain temperature and heart rate data for a particular patient over a certain time period. To obtain this information, the user may, at a user terminal, communicate his desire to the Repository. The Repository, in response, scans the meta-data, and identifies the user, the identity of the Data Cluster in which the desired information is stored, as well as ranges for the data values, if applicable. The user can then query over the network, the particular Data Cluster. In response to such queries, the queried Data Cluster returns the requested data to the user at his terminal. The user terminal may be for example a desktop computer hardwired to the network, or may be a wireless handheld computer, or some other conventional device.

With the above described architecture, standard procedures and protocols for medical data are applied to the data and communication of the same so that requisite privacy, security and integrity are maintained. A principal advantage of the system and method of the invention, is the establishment of a distributed database that permits easy, fast and efficient queries for clinical data in a critical care and/or more general hospital environment, particularly when compared with clinical data systems of the prior art.

### DESCRIPTION OF THE DRAWINGS

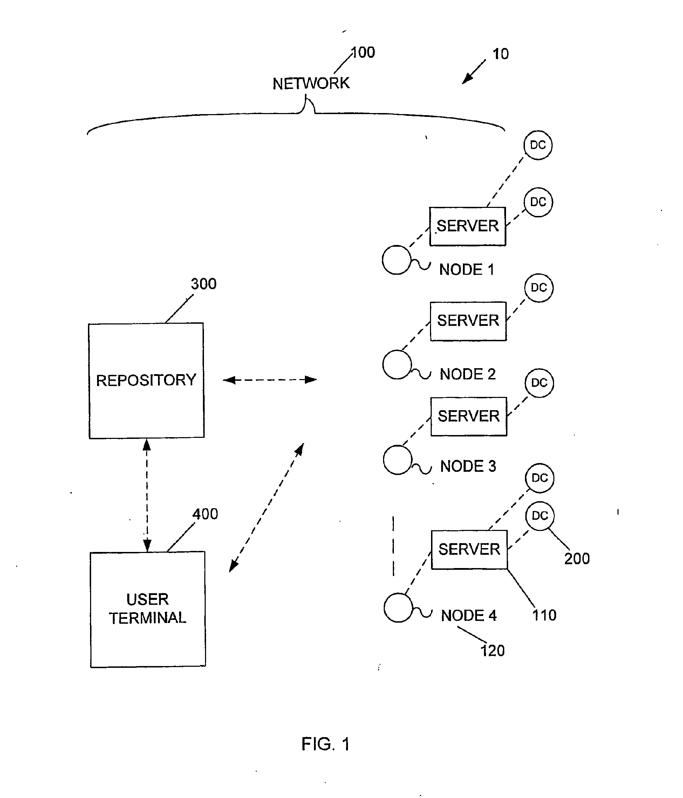

Figure 1 shows, in block diagram form, a clinical data system in accordance with the invention.

### DESCRIPTION OF THE PREFERRED EMBODIMENT

A clinical data system 10 of the present invention is shown in Figure 1. The system and method of the invention establishes a data base architecture that is particularly suited for entry, storage, management and access for clinical data in a critical care and/or more general hospital environment. The system and method of the invention is preferably operative over a geographically distributed network 100 including multiple servers 110, data stores 300 and user access terminals 400, but could alternatively be operative over a local area network. In one form, communication between network nodes 120 can be effected over the internet.

A Data Cluster 200 is a categorized storage location, that holds information for certain sets of categories (that is, patients). A master index establishes a patient-cluster relationship. The system/method directs clinical data flow for each patient to his/her respective Data Cluster. The content of the Data Cluster is described in an external file - referred to as the Repository 300. The Repository 300 includes meta-data that describes the content of all the Data Clusters. This Data Cluster-Repository approach allows a reduction in storage requirements and is easier to maintain, compared to prior art approaches. The Data Cluster size can be selected, based on various parameters such as: maximum number of patients, registration date, and the like. Each Data Cluster 200 is classified by a status, "Active" or "Non-Active". Once a Data Cluster 200 reaches its pre-defined limit, it changes status from Active(wherein it may accept new patients) to Non-Active (wherein it may not accept new patients), and a new Data Cluster is created with the status Active. Several Data Clusters can be present on a single server in a multi-server environment, wherein one of the Data Clusters on that single server is Active. By establishing predetermined fixed sizes to the Data Clusters, the storage loading on the various servers in the multi-server environment can be balanced between the Active Data Clusters on the various servers. This architecture allows system administrator to configure a solution that best fits the hardware capabilities.

In a preferred form of the invention, the various components have the following form:

### Repository:

### Description:

The Repository 300 is a single hospital-wide database that enables users of the system to locate and understand the clinical data in the patient-centric Data Clusters.

The repository mainly includes Meta-Data, principally in the form of data representative of patient identifications and characteristics of the clinical data.

Clinical data collected from different sources (medical devices / hospital systems / medical communications devices) and different locations (hospital units) all are associated with the same repository. With this approach, the disparate patient data are available (i.e., accessible) and understandable (i.e., in an expected, and usually standard, form) regardless of the Data Cluster they are in, while keeping the "size" of the Data Clusters size relatively small (preferably, the Data Clusters may only contain pointers to the Meta-Data in the Repository, and not the Meta-Data itself).

### Repository Content:

Cluster Index - A list of all Data Clusters in the system and the physical locations of the respective Clusters.

Patient Master Index - Data representative of the particular Data Clusters on which the data for the respective patients are resident. The Patient Master Index data supports the EMPI (Enterprise master patient index) standard.

User and Permissions Table - Data associated with the system users (consistent with the HIPPA requirements).

Meta-Data - Data defining all the clinical data resources and their characteristics. For example: the 'Heart Rate' resource has the 'Normal Values Range' characteristic (can accept value between 50-180) and the identification number - 576.

### Data Cluster:

### Description:

The Data Cluster 200 is a data store where the patient clinical data is saved.

Each Data Cluster 200 can support a customized number of patients, consistent with the hardware.

Data in the Data Cluster, that is collected from various sources, supports the HL7 standards.

All of the data for a single patient resides in a single cluster (a single patient's data can not be present in more than a single Data Cluster).

### Content:

● Patient identifier
● Admissions list
● Demographic data (e.g.: patient name, address)
● Diagnosis (e.g.: problem list, ICD 9 /10)
● Vital signs data (e.g.: temperature, blood pressure, heart rate, etc)
● Analog wave data (e.g.: ECG)
● Running orders
● Care plans
● Any other clinical data

### Workflow example:

Steps for locating patient clinical data:
A user wants to check if the Body Temperature value of patient 'John Doe' at 11:15 PM was out of the normal range.
   1) User issues Query to the Repository's 'Patient Master Index' to identify the Data Cluster upon which 'John Doe' data resides.
   2) User issues Query to the Repository's 'Cluster Index' to identify the physical location of the identified Data Cluster.
   3) User issues Query to the Repository's Vital Signs Meta-data to determine the 'HR' identification and Normal Values Range'.
   4) User locates the identified Data Cluster as described in the Repository's 'Cluster Index'.
   5) User issues Query to the located Data Cluster, requesting the Vital Signs patient data, using the Repository's patient identifier and the 'HR' identification.
   6) User receives the requested patient data value, as sent by the Data Cluster, and Compares the retrieved value with the 'Normal Values Range' meta-data that was retrieved from the Repository.

### Medical standards and regulations:

Data residing both in the Repository and in the Data Clusters meet with the following standards and regulations.
● EMPI Standard (Enterprise master patient index)
● HL7 - Health care messaging protocol
● HIPPA - Permissions regulations for discloser of clinical data
● ICD 9 / 10 - A diagnosis coding convention
● CPT - Clinical Path Ways standards

In other forms of the invention, different configurations are used.

Those of skill in the art will recognize that the invention may be embodies in other specific forms without departing from the spirit or essential characteristics thereof. The presently described embodiments are therefore to be considered in all respects as illustrative and not restrictive, the scope of the invention being indicated by the appended claims rather than by the foregoing description, and all variations of the invention which are encompassed within the meaning and range of equivalency of the claims are therefore intended to be embraced therein.

## Claims

1. A system comprising:
one or more servers, each server including one or more data clusters, the data clusters storing patient information including for each patient values associated with clinical data resources;
wherein all the values for a patient are stored in a single data cluster;
a cluster index identifying a server location of each of the data clusters; and
a patient master index including information associating each patient with one of the data clusters.

2. The system of claim 1, further comprising:
a meta data repository describing characteristics of clinical data resources.

3. The system of claim 2, wherein the characteristics of clinical data resources includes a range of normal values.

4. The system of claim 2, further comprising:
a patient information locator configured to:
receive a request for a value of a clinical data resource for a patient;
access the patient master index to identify a data cluster of the data clusters, wherein the identified data cluster stores patient information for the patient;
access the cluster index to identify a server location for the identified data cluster;
access the meta data repository to obtain a resource identifier for the clinical data resource; and
obtain the value from the identified data cluster using the server location and the resource identifier.

5. The system of claim 1, wherein each data cluster includes an indicator identifying if the data cluster can accept an additional patient and corresponding patient information.

6. The system of claim 1, wherein patient information includes a patient identifier, demographic data, a diagnosis, vital signs, and running orders.

7. The system of claim 1, further comprising:
a data collector that collects patient information from at least one of medical devices, hospital systems, and medical communication devices.

8. The system of claim 7, wherein the data collector further collects data from different hospital units.

9. A computer implemented method comprising:
receiving a request from a user for a value of a clinical data resource for a patient;
identifying a single data cluster storing patient information for the patient;
identifying a server location for the identified data cluster, the service location identifying a server that includes the data cluster;
obtaining a resource identifier for the clinical data resource;
obtaining the value from the identified data cluster using the server location and the resource identifier; and
providing the value to the user.

10. The method of claim 9 further comprising:
obtaining a normal value range for the clinical data resource; and
providing the normal value range to the user.

11. The method of claim 9 wherein patient information includes a patient identifier, demographic data, a diagnosis, vital signs, and running orders.
